# EUROPEAN PATENT SPECIFICATION

(11) **EP 3 510 984 B1**
(45) Date of publication and mention of the grant of the patent: **26.08.2020**
(21) Application number: 17863539.7
(22) Date of filing: 29.09.2017
(51) Int. Cl.: A61F 13/56, A61F 13/58

(54) **DISPOSABLE DIAPER**
WEGWERFWINDEL
COUCHE JETABLE

(30) Priority: 31.10.2016 JP 2016212957; 29.11.2016 JP 2016231816
(43) Date of publication of application: 17.07.2019
(73) Proprietor: Unicharm Corporation, Shikokuchuo-shi, Ehime 799-0111 (JP)
(72) Inventor: SAKAGUCHI, Satoru, Kanonji-shi Kagawa 769-1602 (JP); YAMANAKA, Yasuhiro, Kanonji-shi Kagawa 769-1602 (JP); TSUJII, Maki, Kanonji-shi Kagawa 769-1602 (JP)
(74) Representative: Dolleymores
(86) International application number: PCT/JP2017/035516
(87) International publication number: WO 2018/079189

(56) References cited:
- WO-A1-2005/060910
- WO-A1-2015/182179
- JP-A- 2005 537 039
- JP-B2- 5 282 020
- US-A1- 2005 059 950

## Description

### [Technical Field]

The present invention relates to a disposable diaper.

### [Background Art]

Tape-type disposable diapers have conventionally been widely used. In tape-type disposable diapers, pieces of fastening tape are provided on side flaps, and the front portion (the portion on the stomach side, which will also be called the front waist portion) is provided with a piece of target tape for engaging with the fastening tape. For example, PTL 1 discloses a disposable diaper in which the strength of the joining portions between the pieces of fastening tape and the side flaps is reduced such that the pieces of fastening tape can be easily separated from the side flaps.

### [Citation List]

### [Patent Literature]

[PTL 1] Japanese Patent No. 5282020
WO 2015/182179 A1 discloses a disposable diaper for a child weighing 3000g or less, comprising: side flaps, a pair of fastening tapes provided on the side flaps, and a target region. The pair of fastening tapes can be fastened by overlaying one of the fastening tapes on the other fastening tape which is fastened to the target region.

### [Summary of Invention]

### [Technical Problem]

According to the disposable diaper (hereinafter, also simply called the "diaper") of Patent Document 1, even if the wearer of the diaper is in the prone posture for example, the worn diaper can be easily taken off by separating (tearing away) the pieces of fastening tape from the side flaps . However, because the strength of the joining portions between the pieces of fastening tape and the side flaps is weak in the diaper of Patent Document 1, there is a risk that while the diaper is being worn, the pieces of fastening tape will become detached from the side flaps simply due to the wearer moving their body. There is also a risk that the pieces of fastening tape will become detached from the side flaps simply due to the pieces of fastening tape being pulled in order to put the diaper on the wearer.

The present invention was achieved in light of foregoing problems such as that described above, and an aspect of the present invention is to provide a disposable diaper in which the pieces of fastening tape are not likely to become detached when the diaper is worn, and in which the pieces of fastening tape can be easily separated from the side flaps when the diaper is to be taken off.

### [Solution to Problem]

A main aspect of the present invention for achieving the above-described aspect is a disposable diaper having a longitudinal direction, a width direction, and a thickness direction that intersect each other,
the disposable diaper including:
side flaps;
pieces of fastening tape respectively arranged on widthwise side portions of the side flaps; and
joining portions each of which joins a portion of each piece of the fastening tape to a non-skin side of the side flaps in the thickness direction,
in an unfolded state, for each of the pieces of fastening tape,
   a magnitude of force applied at a time when the joining portion is peeled off when a region of the fastening tape that is outward in the width direction with respect to the joining portion is pulled in an outward direction along the width direction
   being greater than
   a minimum value of a magnitude of force applied at a time when the joining portion is peeled off when a region of the fastening tape that is inward in the width direction with respect to the joining portion is pulled in any direction between the outward direction along the width direction and an outward direction along the thickness direction.

Features of the present invention other than the above will become clear by reading the description of the present specification with reference to the accompanying drawings.

### [Advantageous Effects of Invention]

According to the present invention, it is possible to provide a disposable diaper in which pieces of fastening tape are not likely to become detached when the diaper is worn, and in which the pieces of fastening tape can be easily detached from side flaps when the diaper is to be taken off.

### [Brief Description of the Drawings]

FIG. 1 is a plan view showing a state in which a diaper 1 is unfolded and stretched.
FIG. 2A is an exploded illustrative view of a back portion 7 of the diaper 1. FIG. 2B is an exploded illustrative view of a crotch portion 5 of the diaper 1.
FIG. 3 is an illustrative view of a positioning posture.
FIG. 4 is a diagram illustrating a configuration of a piece of fastening tape 30.
FIG. 5 is a plan view illustrating an example of a joining portion 40.
FIGS. 6A to 6C are cross-sectional schematic views illustrating a method for taking off the diaper 1 by peeling off joining portions 40.
FIGS. 7A and 7B are diagrams illustrating force acting on a joining portion 40.
FIG. 8 is a plan view illustrating a variation of the joining portion 40.
FIG. 9 is a diagram illustrating force acting on the joining portion 40 when a piece of fastening tape 30 is pulled in the longitudinal direction of the diaper 1.
FIG. 10 is a plan view showing another variation of the joining portion 40.
FIG. 11 is a plan view showing a variation (diaper 2) of the diaper 1 in an unfolded and stretched state.
FIG. 12 is an exploded illustrative view of the back portion 7 of the diaper 2.

### [Description of Embodiments]

At least the following matters will become clear with the description of this specification and the attached drawings. A disposable diaper having a longitudinal direction, a width direction, and a thickness direction that intersect each other,
the disposable diaper including:
side flaps;
pieces of fastening tape respectively arranged on widthwise side portions of the side flaps; and
joining portions each of which joins a portion of each piece of the fastening tape to a non-skin side of the side flaps in the thickness direction,
in an unfolded state, for each of the pieces of fastening tape,
a magnitude of force applied at a time when the joining portion is peeled off when a region of the fastening tape that is outward in the width direction with respect to the joining portion is pulled in an outward direction along the width direction
being greater than
a minimum value of a magnitude of force applied at a time when the joining portion is peeled off when a region of the fastening tape that is inward in the width direction with respect to the joining portion is pulled in any direction between the outward direction along the width direction and an outward direction along the thickness direction.

According to this disposable diaper, spontaneous detachment of the pieces of fastening tape due to being pulled in the worn state of the diaper is suppressed, and the pieces of fastening tape can be easily detached from the side flaps when removing the diaper.

In such a disposable diaper, it is desirable that
in the unfolded state, for each of the pieces of fastening tape,
the magnitude of force applied at the time when the joining portion is peeled off when the region of the fastening tape that is outward in the width direction with respect to the joining portion is pulled in the outward direction along the width direction
is greater than
a maximum value of the magnitude of force applied at the time when the joining portion is peeled off when the region of the fastening tape that is inward in the width direction with respect to the joining portion is pulled in any direction between the outward direction along the width direction and the outward direction along the thickness direction.

According to this disposable diaper, when the diaper in the worn state is to be taken off, the pieces of fastening tape can be easily detached from the side flaps regardless of the direction in which the pieces of fastening tape are pulled. Accordingly, the operation of taking off the diaper is easier, and it is possible to further reduce the burden on the wearer's body.

In such a disposable diaper, it is desirable that
for each of the pieces of fastening tape,
a widthwise stretchability in a widthwise region of the fastening tape that has a predetermined width and that includes the joining portion is less than or equal to
a widthwise stretchability in a region of the fastening tape other than the widthwise region that has the predetermined width.

According to this disposable diaper, even if the pieces of fastening tape are pulled in the width direction in an operation for putting on or taking off the diaper, the regions provided with the joining portions are not likely to stretch/contract, and therefore the joining portions are not likely to deform, and the joining strength is not likely to decrease. Accordingly, even if the wearer moves their body while wearing the diaper, it is possible to suppress the case where the joining portions become spontaneously peeled off.

In such a disposable diaper, it is desirable that
the widthwise region of the fastening tape that has the predetermined width has no widthwise stretchability.

According to this disposable diaper, when performing an operation for peeling off the joining portions by pulling the pieces of fastening tape, the force pulling the pieces of fastening tape is likely to be effectively applied to the joining portions. Accordingly, the joining portions can be more easily peeled off with a smaller magnitude of force.

In such a disposable diaper, it is desirable
that each of the joining portions has a plurality of dot-like joining portions that are arranged intermittently along the width direction, and
that an area of a dot-like joining portion arranged at a position that is farthest inward in the width direction is smaller than an area of a dot-like joining portion arranged at another position.

According to this disposable diaper, when performing an operation for peeling off the joining portions by pulling the pieces of fastening tape in the width direction, the joining strength of the dot-like joining portions arranged in the region that is first subjected to the pulling force is weaker than the joining strength of the dot-like joining portions arranged in the other region, thus making it easier to peel off the pieces of fastening tape.

In such a disposable diaper, it is desirable that
in the unfolded state, for each of the pieces of fastening tape,
the magnitude of force applied at a time when the joining portion is peeled off when the region of the fastening tape that is outward in the width direction with respect to the joining portion is pulled in the outward direction along the width direction
is greater than
a minimum value of a magnitude of force applied at a time when the joining portion is peeled off when the region of the fastening tape that is inward in the width direction with respect to the joining portion is pulled from a waist side toward a crotch side along the longitudinal direction.

According to this disposable diaper, spontaneous detachment of the pieces of fastening tape due to being pulled in the worn state of the diaper is suppressed, and, when removing the diaper, the pieces of fastening tape can be easily detached from the side flaps even if the pieces of fastening tape are pulled along the longitudinal direction.

In such a disposable diaper, it is desirable
that each of the joining portions has a plurality of dot-like joining portions that are arranged intermittently along the longitudinal direction, and
that an area of a dot-like joining portion arranged at a position that is farthest on the waist side in the longitudinal direction is smaller than an area of a dot-like joining portion arranged at another position.

According to this disposable diaper, when performing an operation for peeling off the joining portions by pulling the pieces of fastening tape in the longitudinal direction, the joining strength of the dot-like joining portions arranged in the region that is first subjected to the pulling force is weaker than the joining strength of the dot-like joining portions arranged in the other region, thus making it easier to peel off the pieces of fastening tape.

In such a disposable diaper, it is desirable that
for each of the pieces of fastening tape,
the magnitude of force applied at a time when the joining portion is peeled off when the region of the fastening tape that is inward in the width direction with respect to the joining portion is pulled from the waist side toward the crotch side along the longitudinal direction is smaller than
a magnitude of force applied to peel off the joining portion when the region of the fastening tape that is inward in the width direction with respect to the joining portion is pulled from the crotch side toward the waist side along the longitudinal direction.

According to this disposable diaper, it is possible to suppress problems such as the joining portions being peeled off from the crotch side toward the waist side and the diaper spontaneously coming off when a low-weight infant who is wearing the diaper and is in a positioning posture moves their legs. However, when the diaper is to be taken off, the joining portions can be easily peeled off by inserting a finger into the diaper and then pulling the pieces of fastening tape in the direction from the waist side toward the crotch side.

In such a disposable diaper, it is desirable that
for each of the pieces of fastening tape,
a longitudinal distance from a waist-side end of the side flap to a waist-side end of the fastening tape
is longer than
a widthwise distance from an inward end of the fastening tape to an inward end of the joining portion.

According to this disposable diaper, when performing an operation for peeling off the joining portions by pulling the pieces of fastening tape, a worker can easily use one hand to grab the longitudinal waist-side end portion of a side flap, and easily use another hand to grab the widthwise inward end portion of a piece of fastening tape. Accordingly, the force pulling the piece of fastening tape can be effectively applied to the joining portion.

In such a disposable diaper, it is desirable
that the disposable diaper further comprises a target region to which the pieces of fastening tape are to be engaged, and
that in the unfolded state, for each of the pieces of fastening tape,
   the magnitude of force applied at the time when the joining portion is peeled off when the region of the fastening tape that is inward in the width direction with respect to the joining portion is pulled in any direction between the outward direction along the width direction and the outward direction along the thickness direction
   is greater than
   a magnitude of force applied at a time when the fastening tape is detached from the target region in a state where the fastening tape is engaged with the target region.

According to this disposable diaper, the pieces of fastening tape can be more easily repeatedly attached to and detached from the target region, and it is easier to adjust the fit around the waist and the worn position of the diaper 1. Also, the pieces of fastening tape and the side flaps can be joined such that they are not easily detached from each other.

In such a disposable diaper, it is desirable that
an indicator mark is provided that indicates the region of the fastening tape that is inward in the width direction with respect to the joining portion.

According to this disposable diaper, when the diaper 1 in the worn state is to be taken off, the worker can more easily and accurately recognize which portion of the fastening tape is to be pulled in order to easily peel off the joining portion.

A disposable diaper having a longitudinal direction, a width direction, and a thickness direction that intersect each other,
the disposable diaper including:
a liquid-absorbent absorbent body;
an exterior sheet provided on a non-skin side in the thickness direction with respect to the absorbent body;
pieces of fastening tape respectively arranged on widthwise side portions of the exterior sheet; and
joining portions each of which joins a portion of each piece of the fastening tape to a non-skin side of the exterior sheet in the thickness direction,
in an unfolded state, for each of the pieces of fastening tape,
   a magnitude of force applied at a time when the joining portion is peeled off when a region of the fastening tape that is outward in the width direction with respect to the joining portion is pulled in an outward direction along the width direction
   being greater than
   a minimum value of a magnitude of force applied at a time when the joining portion is peeled off when a region of the fastening tape that is inward in the width direction with respect to the joining portion is pulled in any direction between the outward direction along the width direction and an outward direction along the thickness direction.

According to this disposable diaper, spontaneous detachment of the pieces of fastening tape due to being pulled in the worn state of the diaper is suppressed, and the pieces of fastening tape can be easily detached from the exterior sheet when removing the diaper.

### Embodiments

### Basic configuration of disposable diaper 1

A disposable diaper 1 (hereinafter, also simply called the "diaper 1") of the present embodiment is a disposable diaper mainly for being worn by a newborn infant or an infant, and is favorably used with a low-weight infant having a body weight of 3,000 g or less, and particularly with a low-birth-weight infant having a body weight of 2,500 g or less. Note that the concept of "low-weight infant" includes not only a low-birth-weight infant (body weight less than 2,500 g), but also a very-low-birth-weight infant (body weight less than 1,500 g) and an extremely-low-birth-weight infant (body weight less than 1,000 g).

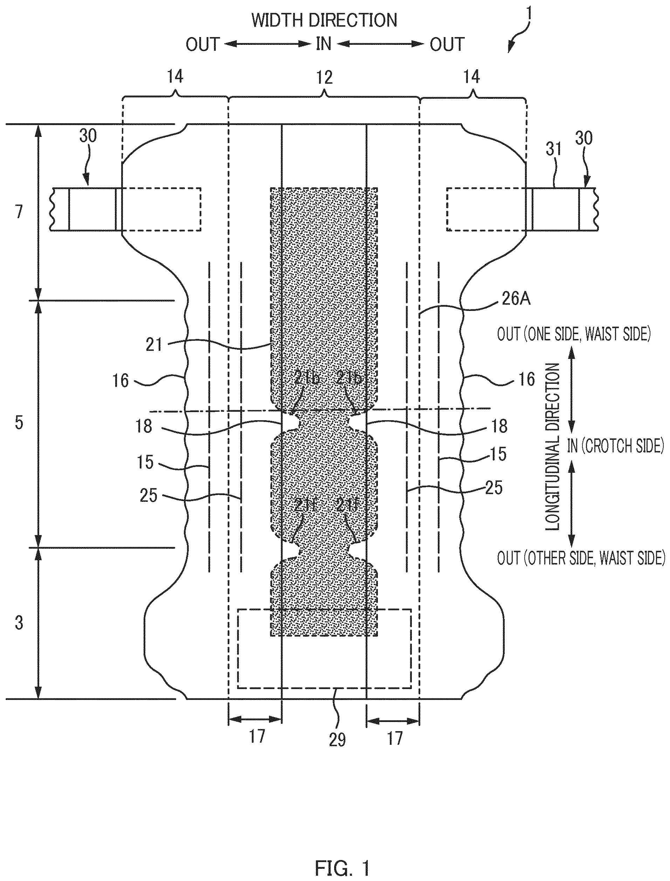

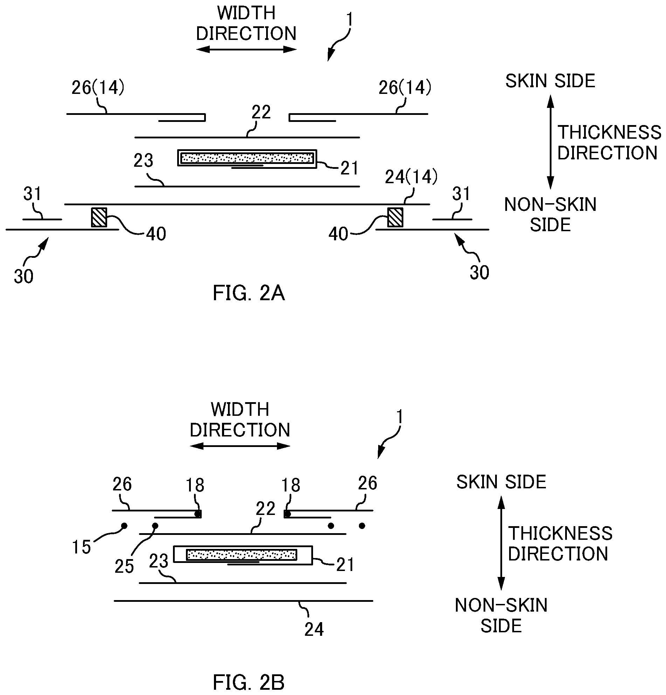

FIG. 1 is a plan view showing a state in which the diaper 1 is unfolded and stretched. FIG. 2A is an exploded illustrative view of a back portion 7 of the diaper 1. FIG. 2B is an exploded illustrative view of a crotch portion 5 of the diaper 1. Note that the state where the diaper 1 has been stretched refers to a state in which the diaper 1 has been stretched to the extent that wrinkles formed in the diaper 1 can substantially no longer be seen when the diaper 1 in the unfolded state, or in other words a state in which the diaper 1 is stretched such that the lengths of the constituent members of the diaper 1 (e.g., a later-described top sheet 22) match or are close to the dimensions of the members on their own.

The diaper 1 of the present embodiment is a so-called tape-type disposable diaper, and as shown in FIG. 1, has a front portion 3, a crotch portion 5, and a back portion 7. The front portion 3 is the portion that is located at the front side of the wearer (stomach side, front waist region) . Also, the back portion 7 is the portion that is located at the back side of the wearer (back side, back waist region) . The crotch portion 5 is the portion that is located between the front portion 3 and the back portion 7.

As shown in FIG. 1, various directions used in the following description are defined as follows. The direction from the front portion 3 toward the back portion 7 is the "longitudinal direction", and a direction orthogonal to the longitudinal direction is the "width direction". CL shown in FIG. 1 is a line that indicates the center of the diaper 1 in the longitudinal direction. Also, as shown in FIG. 2, a direction orthogonal to the longitudinal direction and the width direction is the "thickness direction", the side corresponding to the wearer's skin is the "skin side", and the side opposite thereto is the "non-skin side".

The diaper 1 has a central band-shaped region 12, side flaps 14, leg gathers 16, and leg side gathers 17. Pieces of fastening tape 30 are respectively attached to the front portion 3 of the pair of side flaps 14.

The central band-shaped region 12 is a band-shaped region that is located in the central portion in the width direction that is constituted by the front portion 3, the crotch portion 5, and the back portion 7. The central band-shaped region 12 is a portion that absorbs and holds a liquid such as urine that is excreted by the wearer. The central band-shaped region 12 has a longitudinally elongated shape (shape extending in the longitudinal direction) that includes a liquid-holding absorbent body 21. The central band-shaped region 12 mainly has the absorbent body 21, a top sheet 22, a leak-proof sheet 23, and a back sheet 24 (see FIGS. 2A and 2B).

The absorbent body 21 is a member obtained by overlaying sheets of liquid absorbent material that can absorb excrement such as urine, and is arranged extending over the front portion 3, the crotch portion 5, and the back portion 7. The shaded region in FIG. 1 indicates the region occupied by the absorbent body 21. The absorbent body 21 of the present embodiment is approximately shaped as a rectangle that is elongated in the longitudinal direction. It should be noted that the shape of the absorbent body 21 is not limited to the shape shown in FIG. 1. Also, it is sufficient that the absorbent body 21 is provided in at least the crotch portion 5. The absorbent body 21 is arranged so as to be sandwiched between the top sheet 22 and the leak-proof sheet 23. The liquid absorbent material that constitutes the absorbent body 21 can be liquid-absorbent fibers such as pulp fibers or a liquid-absorbent particulate matter such as a superabsorbent polymer, for example. A liquid absorbent material other than liquid-absorbent fibers and liquid-absorbent particulate matter may also be included.

One pair of dart portions 21f are formed in the crotch portion 5 of the absorbent body 21 at positions close to the front portion 3 in the two widthwise end portions. Similarly, one pair of dart portions 21b are formed at positions close to the back portion 7 in the two widthwise end portions. The dart portions 21f and 21b are narrow portions formed by cutting out portions of the absorbent body 21 in an approximately bell shape as shown in FIG. 1. Note that the shapes of the dart portions 21f and 21b are not limited to the shapes shown in FIG. 1. Also, instead of being formed as cutouts in the absorbent body 21, the dart portions 21f and 21b may be formed by reducing the base weight of the liquid absorbent material compared to other portions of the absorbent body 21. For example, the base weight of the liquid absorbent material in the dart portions 21f and 21b may be set to less than or equal to 1/3 the base weight in the other portions. In any case, the stiffness of the absorbent body 21 is lower in the dart portions 21f and 21b than in the other portions. Accordingly, the absorbent body 21 can bend more easily in the dart portion regions, and the diaper 1 can more easily deform into a three-dimensional round shape to fit the wearer's body (crotch portion), thus making it possible to improve the fit when the diaper 1 is worn.

The top sheet 22 is a liquid-permeable member that is arranged on the skin side of the absorbent body 21. The leak-proof sheet 23 is a liquid-impermeable member that is arranged on the non-skin side of the absorbent body 21. The back sheet 24 is a member that configures the exterior on the non-skin side of the diaper 1 (i.e., is an exterior sheet), and is constituted by nonwoven fabric for example. The back sheet 24 is arranged on the non-skin side of the leak-proof sheet 23 (see FIGS. 2A and 2B).

A pair of leg elastic members 25 (e.g., elastic strings) that can stretch and contract in the longitudinal direction are provided between the absorbent body 21 and the back sheet 24 in at least the crotch portion 5 in the central band-shaped region 12. The leg elastic members 25 are members that give stretchability to the central band-shaped region 12 in the crotch portion 5. In the present embodiment, the leg elastic members 25 are attached in a state of being stretched in the longitudinal direction. Accordingly, the leg elastic member 25 applies contractive force acting in the longitudinal direction to the crotch portion 5 of the central band-shaped region 12. When the diaper 1 is put on, the crotch portion 5 contracts in the longitudinal direction, thus, along with the above-described dart portions 21f and 21b, allowing the central band-shaped region 12 to deform into a three-dimensional rounded shape conforming to the wearer's body. Accordingly, the central band-shaped region 12 is held in a shape that surrounds the wearer's crotch portion, thus improving the fit of the diaper 1 while also making it easier to suppress the leakage of excrement to the outside of the diaper 1.

The side flaps 14 are portions located on the two widthwise side portions of the central band-shaped region 12. The side flaps 14 are formed extending over the front portion 3, the crotch portion 5, and the back portion 7 (see FIG. 1). The widthwise length (width) of the side flaps 14 in the crotch portion 5 is smaller than the widthwise length (width) of the side flaps 14 in the front portion 3 and the back portion 7. The side flaps 14 are mainly constituted by a skin-side sheet 26 and the back sheet 24 (see FIGS. 2A and 2B). The skin-side sheet 26 is a skin-side member that is formed extending over the front portion 3, the crotch portion 5, and the back portion 7, and is constituted by nonwoven fabric for example. The skin-side sheet 26 is a member that constitutes the leg side gathers 17 (barrier cuffs), and the outward portions of the skin-side sheet 26 (the portions outward of a joining portion 26A shown by dashed lines in FIG. 1) constitute the side flaps 14.

A leg-gather elastic member 15 that stretches and contracts in the longitudinal direction is provided in each of the two side flaps 14. The leg-gather elastic members 15 are elastic members such as elastic strings that stretch and contract in the longitudinal direction, and are members that give stretchability to the leg openings when the diaper 1 is worn. Specifically, the leg-gather elastic members 15 are leg elastic members that allow the leg portions of the diaper 1 to fit to the legs of the wearer. Also, the leg-gather elastic members 15 give stretchability to the skin-side sheet 26 and the back sheet 24 of the crotch portion 5, thus constituting the leg gathers 16.

The leg side gathers 17 are barrier cuffs for preventing the leakage of liquids through gaps around the legs. The pair of leg side gathers 17 extend in the longitudinal direction over the front portion 3, the crotch portion 5, and the back portion 7 (see FIG. 1). The leg side gathers 17 are formed so as to cover the two edges of the central band-shaped region 12 on the inward sides of the side flaps 14.

The leg side gathers 17 (barrier cuffs) are mainly constituted by portions of the skin-side sheet 26 that are on the inward side in the width direction (see FIG. 2). The inner edges of the skin-side sheet 26 of the crotch portion 5 have stretchability due to leg-side-gather elastic members 18 such as elastic strings. The skin-side sheet 26 is joined along the longitudinal direction in the joining portion 26A that is between the central band-shaped region 12 and the side flap 14. When the diaper 1 is worn, due to the stretchability of the leg-side-gather elastic members 18, the region inward of the joining portion 26A in the skin-side sheet 26 rises toward the wearer's skin at the joining portion 26A, thus suppressing the lateral leakage of excrement or the like.

The pieces of fastening tape 30 are arranged at the two widthwise side portions of the side flaps 14 in the back portion 7 of the diaper 1 (see FIG. 1). The pieces of fastening tape 30 of the present embodiment each include an engaging portion 31 that is constituted by a hook-and-loop fastener or the like, and the engaging portion 31 can be engaged with later-described target tape 29. When the diaper 1 is to be put on the wearer (infant or the like), the pieces of fastening tape 30 (engaging portions 31) are engaged with the target tape 29, thus forming the waist opening and the leg openings of the diaper 1, and making it possible to fix the position of the diaper 1 relative to the wearer's body (trunk) . For each piece of fastening tape 30, the region of a portion of the skin-side surface is joined to the non-skin side of the side flap 14 (back sheet 24) via a joining portion 40. Details of the pieces of fastening tape 30 and the joining portions 40 will be described later.

The target tape 29 is provided in the front portion 3 of the central band-shaped region 12 (see FIG. 1). The target tape 29 is arranged on the non-skin side of the back sheet 24 of the front portion 3. The target tape 29 is a member that can engage with the engaging portions 31 of the pieces of fastening tape 30, and are formed by nonwoven fabric for example. The target tape 29 configures a target region for engagement with the pieces of fastening tape 30. Note that instead of the target tape 29 being arranged on the non-skin side of the back sheet 24, the target region may be directly formed on the outermost piece of nonwoven fabric that constitutes the back sheet 24. Also, the diaper 1 is put on by engaging the fastening tape 30 with the pieces of target tape 29.

The product length in the longitudinal direction (dimension in the state where the product is stretched to eliminate wrinkles in the longitudinal direction) of the diaper 1 for a low-weight infant of the present embodiment is in a range of 210 to 330 mm. For example, the product length of the diaper 1 for a low-birth-weight infant having a body weight less than 2,500 g is 310 mm, the product length in the case of the diaper 1 for a very-low-birth-weight infant having a body weight less than 1,500 g is 270 mm, and the product length in the case of the diaper 1 for an extremely-low-birth-weight infant having a body weight less than 1,000 g is 230 mm.

Also, the waist dimension of the diaper 1 for a low-weight infant of the present embodiment is in a range of 160 to 295 mm. Note that the waist dimension is the dimension in the state where the product is stretched to eliminate wrinkles while the end portion of one piece of fastening tape 30 is matched with the end portion on the side flap 14 side of a hook sheet region C of the other piece of fastening tape 30. In other words, it is the dimension in the state where the product is stretched in the width direction. For example, the waist dimension of the diaper 1 for a low-birth-weight infant is 273.5 mm, and the waist dimension of the diaper 1 for a very-low-birth-weight infant having a body weight less than 1,500 g is 220 mm.

### Attachment/removal of diaper 1

In the case of putting on a tape-type disposable diaper such as the diaper 1, in a general method, the two pieces of fastening tape 30 provided on the two widthwise side portions of the back portion 7 of the side flaps 14 are engaged with the target tape 29 provided on the non-skin side of the front portion 3 (stomach side) of the central band-shaped region 12, thus fitting the diaper around the trunk of the wearer.

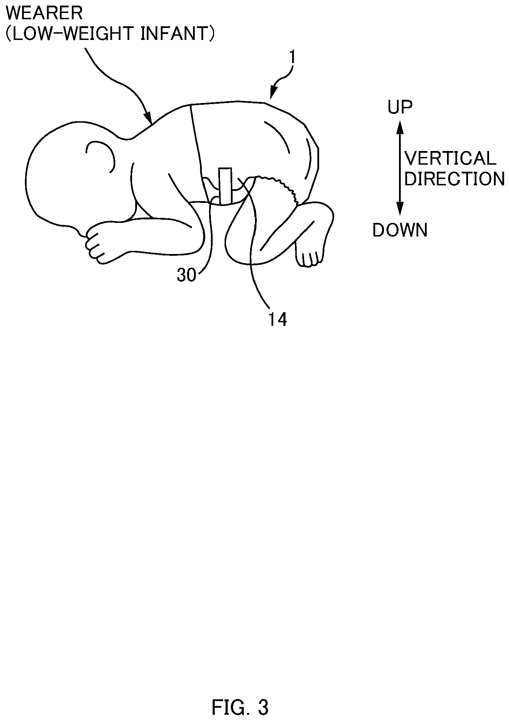

It should be noted that a low-weight infant, who is the target wearer of the diaper 1 of the present embodiment, is generally kept in an incubator in a hospital or the like. For this reason, in order to put on a diaper for a low-weight infant, a worker (person who puts the diaper on a low-weight infant and takes it off) puts their hand through an operation window provided in the incubator and needs to perform the task in a small space. Also, it is common for the low-weight infant to be placed in the incubator in a prone posture in which their back is curved in a C shape and their legs are sharply bent into an M shape, as shown in FIG. 3. This posture will also be called the "positioning posture", which is a posture that is close to the posture taken in the mother's womb (infant-like bent posture) and is a resting posture. When the low-weight infant is in the positioning posture, the engaging portions between the target tape and the pieces of fastening tape are located below the stomach of the wearer (low-weight infant) as shown in FIG. 3.

Accordingly, conventionally, when performing the task of changing the diaper on a low-weight infant that is in the positioning posture in an incubator, the worker has needed to put their hand under the low-weight infant's stomach inside the small space and peel the target tape away from the pieces of fastening tape. This task is complicated for the worker, and there has also been a risk of damaging or burdening the skin of the low-weight infant who is the wearer.

In contrast, with the diaper 1 of the present embodiment, while the target tape 29 and the pieces of fastening tape 30 are engaged, it is possible to peel off joining portions 40 where the pieces of fastening tape 30 and the side flaps 14 are joined. Accordingly, the diaper 1 can be removed without disengaging the target tape 29 from the pieces of fastening tape 30. Accordingly, the diaper 1 can be safely and easily replaced even in the case where the wearer (low-weight infant) is in the positioning posture in the incubator.

### Details of fastening tape 30

The following describes the configuration of each of the pieces of fastening tape 30 that are joined to the side flaps 14 via the joining portions 40, and a method of peeling off the joining portions 40.

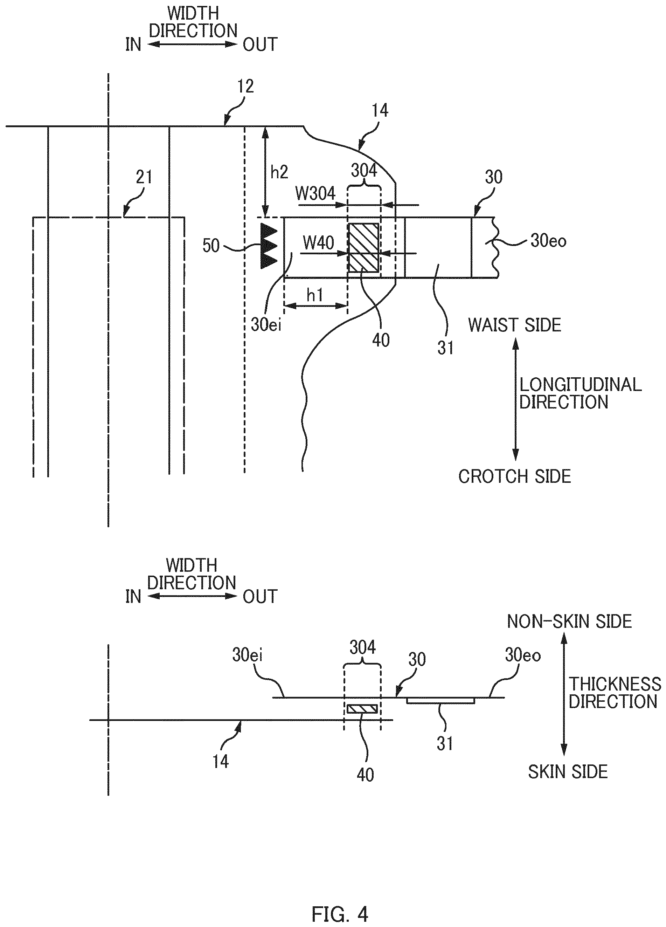

FIG. 4 is a diagram illustrating the configuration of a piece of fastening tape 30. As shown in FIG. 4, the fastening tape 30 is a sheet member that is approximately shaped as a rectangle that is elongated in the width direction. With respect to the width direction, an inward end portion 30ei of the fastening tape 30 has a portion that is overlapped with the skin side in the thickness direction of the side flap 14, and an outward end portion 30eo of the fastening tape 30 has a portion that projects outward from the end of the side flap 14.

In the portion where the fastening tape 30 and the side flap 14 are overlapped in the thickness direction, the joining portion 40 is formed in the range of a region 304 that has a predetermined width W304 in the width direction. The joining portion 40, which is indicated by hatching in FIG. 4, extends in the longitudinal direction and has a predetermined width W40 that is smaller than the width W304 in the width direction (W304>W40) . In the present embodiment, the joining portion 40 is formed using welding through heat sealing, sonic sealing, or the like, adhesion using a hot-melt adhesive or the like, or another joining means, and thus the fastening tape 30 is joined to the non-skin side of the side flap 14. Note that as shown in FIG. 4, in the fastening tape 30 of the present embodiment, a region inward of the region 304 in the width direction (i.e., a predetermined region including the inward end portion 30ei) is a dry edge in which the joining portion 40 is not formed.

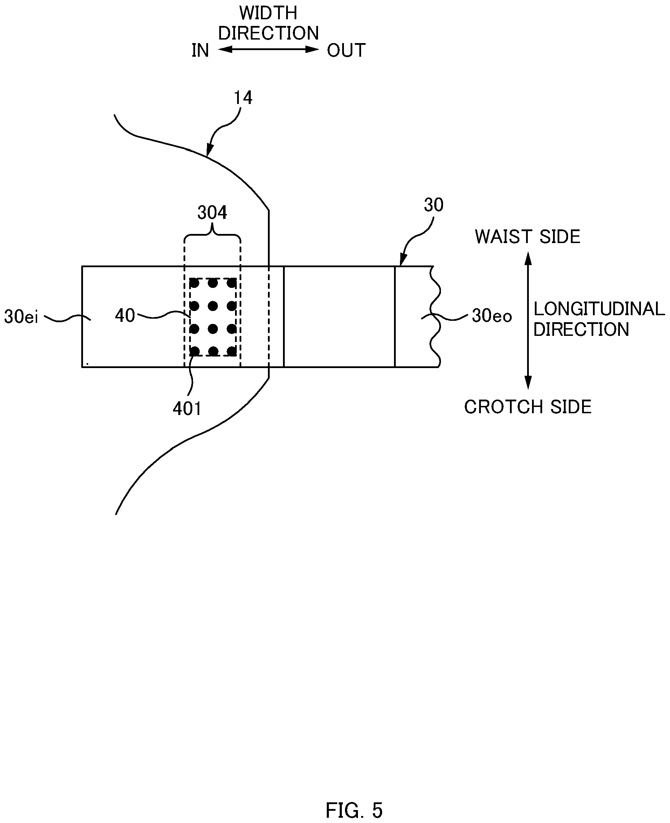

Also, instead of the fastening tape 30 and the side flap 14 being joined in the entirety of the region indicated by hatching in FIG. 4 (i.e., the region indicated by the width W40), the joining portion 40 may be formed such that the fastening tape 30 and the side flap 14 are joined in a portion of the region indicated by hatching. FIG. 5 is a plan view illustrating an example of the joining portion 40. In the example shown in FIG. 5, dot-like joining portions 401 indicated by solid black circles are intermittently formed in the range of the region 304 of the fastening tape 30, with three in each row along the width direction and four in each column in the longitudinal direction, thus forming the joining portion 40. In this way, the joining portion 40 is formed by a plurality of dot-like joining portions 401, thus making it possible to change the joining strength of the fastening tape 30 to the side flap 14. For example, the joining strength in the joining portion 40 can be freely adjusted by changing the size (area) of the individual dot-like joining portions 401 and the arrangement interval thereof.

The engaging portion 31 (see FIG. 4) is formed in a region of the fastening tape 30 that corresponds to a portion of the skin-side surface in the portion that projects outward from the side portion in the width direction of the side flap 14. The engaging portion 31 is constituted by a hook-and-loop fastener that includes a plurality of locking projections (hooks) for example, and these locking projection become caught on loop-shaped fibers in the target tape 29 (nonwoven fabric) provided in the front portion 3, thus engaging these pieces of tape with each other.

Also, the fastening tape 30 of the present embodiment is not likely to stretch and contract in the width direction in at least the range of the region 304 in FIG. 5. In other words, concerning a predetermined region of the fastening tape 30 that includes the widthwise region in which the joining portion 40 is formed, its widthwise stretchability is less than or equal to the widthwise stretchability in the other region. In other words, the stretchability is the lowest in the widthwise region of the fastening tape 30 in which the joining portion 40 is formed. Accordingly, even if the fastening tape 30 is pulled in the width direction when the diaper 1 is put on or taken off, the region in which the joining portion 40 is formed is not likely to stretch, the joining portion 40 is not likely to deform, and therefore the joining strength is not likely to decrease. Accordingly, even if the wearer moves their body while wearing the diaper 1, it is possible to suppress the case where the joining portion 40 becomes spontaneously peeled off.

Furthermore, it is desirable that the range of the region 304 is non-stretchy in the width direction. If this region is non-stretchy, at least the portions adjacent to the two widthwise ends of the joining portion 40 do not stretch or contract in the width direction. Accordingly, when the joining portion 40 is peeled off by pulling the inward end portion 30ei in the width direction of the fastening tape 30 as will be described later, the force (F2) pulling the fastening tape 30 is likely to be efficiently applied to the joining portion 40. In other words, it is possible to suppress the case where the force pulling the fastening tape 30 is canceled by stretching of the fastening tape 30 for example, thus making it easier to peel off the joining portion 40 with a smaller magnitude of force.

Also, it is desirable that the stretchability with respect to the width direction is raised in a region outward of the region 304 in the width direction. Specifically, a configuration is possible in which in the fastening tape 30, the stretchability of the region outward in the width direction of the region where the joining portion 40 is formed is higher than the stretchability of the region in which the joining portion 40 is formed in the width direction. According to this configuration, when the fastening tape 30 is pulled in the width direction in order to put the diaper 1 on a low-weight infant, the side flap 14 is more easily pulled outward and becomes wider in the width direction due to the stretchability of the fastening tape 30 itself, thus suppressing the case where wrinkles are formed at the waist opening of the diaper 1 and the side flap 14 bends. Accordingly, it is possible to improve the fit of the diaper 1.

### Method of peeling off joining portion 40

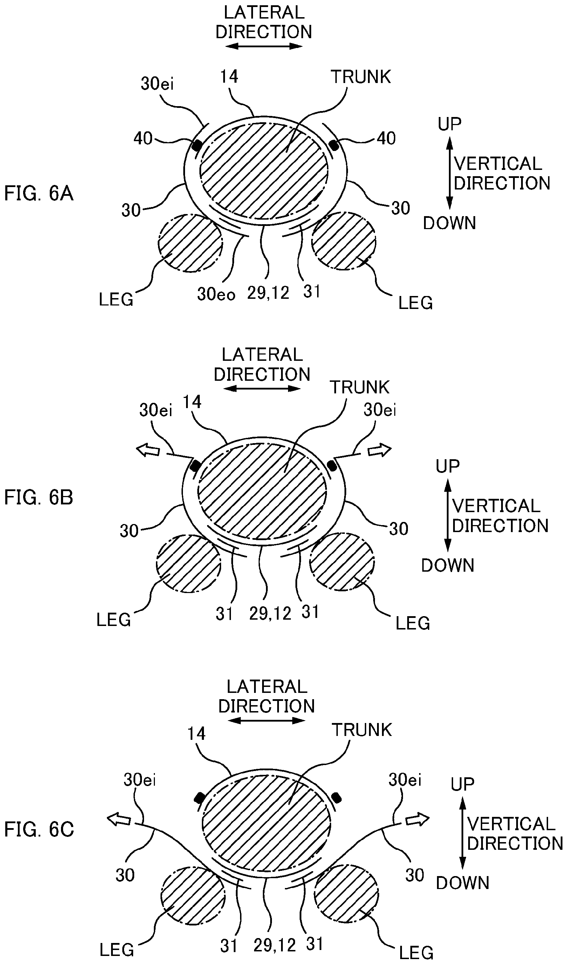

FIGS. 6A to 6C are cross-sectional schematic views illustrating a method for taking off the diaper 1 by peeling off the joining portions 40. FIGS. 6A to 6C show cross-sections of the waist portion of the wearer (low-weight infant) wearing the diaper 1 while in the positioning posture shown in FIG. 3. In the state shown in FIG. 6A, the portions where the engaging portions 31 of the pieces of fastening tape 30 and the target tape 29 are engaged are located below the wearer's stomach, and the wearer's legs are located on respective sides of these engaged portions . In this state, it is difficult to grab and pull laterally the outward end portions 30eo of the pieces of fastening tape 30 to peel them off the target tape 29.

In view of this, as shown in FIG. 6B, the worker grabs and pulls the inward end portions 30ei of the pieces of fastening tape 30 outward in the lateral direction (the arrow directions in FIG. 6B). The inward end portions 30ei are located on the back side of the wearer (low-weight infant), and therefore even if the wearer is in the positioning posture, these portions can be easily seen from the outside, and at least portions of the inward end portions 30ei are dry edges as previously described, thus making it possible for the worker to easily grab the inward end portions 30ei. Note that in FIG. 6B, the worker may pull the inward end portions 30ei downward instead of outward in the lateral direction.

When the force for pulling the inward end portions 30ei rises above the joining force in the joining portions 40, the joining portions 40 become peeled off as shown in FIG. 6C, and the pieces of fastening tape 30 detach from the side flaps 14. Accordingly, the waist opening of the diaper 1 is released from the wearer, and the worker can remove the diaper 1 from under the wearer's crotch.

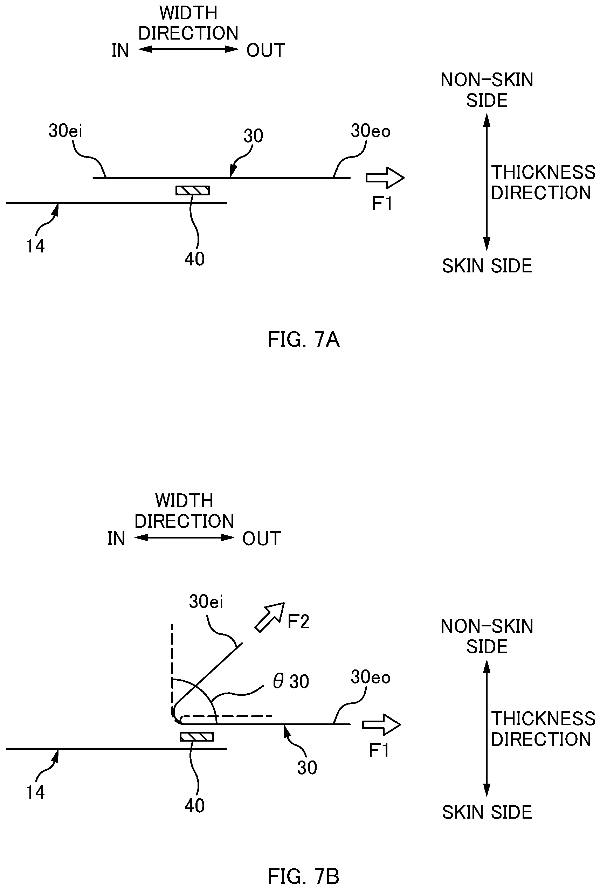

FIGS. 7A and 7B are diagrams illustrating force acting on a joining portion 40. Note that when the diaper 1 is worn, the pieces of fastening tape 30 and the side flaps 14 are curved around the wearer's waist as shown in FIGS. 6A to 6C. In contrast, for the sake of the description, FIGS. 7A and 7B show the piece of fastening tape 30 and the side flap 14 in the unfolded state (flat state).

FIG. 7A is a diagram for illustrating force acting on the joining portion 40 when the diaper 1 is in the normal worn state. In the worn state of the diaper 1 in FIG. 7A, the widthwise outward end portion 30eo of the fastening tape 30 is engaged with the target tape 29 (not shown in FIG. 7A). In order to improve the fit at the wearer's waist, the region of the fastening tape 30 that is outward in the width direction with respect to the joining portion 40 (i.e., the region including the outward end portion 30eo) is engaged with the target tape 29 in a state of being pulled outward in the width direction in FIG. 7A. Accordingly, there are the force by which the fastening tape 30 is pulled outward in the width direction and the resistance force of the side flap 14 that is joined to the fastening tape 30 via the joining portion 40, resulting in the force along the width direction acting on the joining portion 40. Furthermore, in the present embodiment, the fastening tape 30 itself has stretchability in the width direction, and therefore the joining portion 40 is subjected to contracting force of the fastening tape 30 generated due to being pulled and stretched. Accordingly, force F1 acting outward in the width direction is applied to the joining portion 40. It is assumed that when the magnitude of this force F1 reaches F1S, the joining portion 40 is peeled off, and the fastening tape 30 detaches from the side flap 14.

Next, FIG. 7B is a diagram illustrating force that acts on the joining portion 40 when the diaper 1 is removed. As previously described using FIGS. 6A to 6C, when the diaper 1 is to be removed, in order to peel off the joining portions 40, the regions of the pieces of fastening tape 30 that are inward in the width direction with respect to the joining portions 40 (i.e., the inward end portions 30ei) are pulled in a predetermined direction. Here, the "predetermined direction" in which each of the inward end portions 30ei is pulled is, as shown in FIG. 7B, any direction within an angle θ30 that is from the direction that is outward in the width direction with respect to the joining portion 40 to the direction that is outward (toward the non-skin side) in the thickness direction. In other words, the predetermined direction is any direction that is from the outward direction along the width direction to the outward direction along the thickness direction. That is to say, the predetermined direction is a direction in which the inward end portion 30ei can possibly be pulled in the case where the worker attempts to peel the piece of fastening tape 30 off the joining portion 40 in the state where the diaper 1 is worn by a low-weight infant that is sleeping in the positioning posture. In FIG. 7B, a force F2 acting in the "predetermined direction" is acting on the joining portion 40. It is assumed that when the magnitude of this force F2 reaches F2S, the joining portion 40 is peeled off, and the fastening tape 30 detaches from the side flap 14.

In the diaper 1 of the present embodiment, the joining portion 40 is formed such that the force F1S applied at the time when the joining portion 40 is peeled off when pulling the fastening tape 30 in an outward direction along the width direction is smaller than the minimum value of the force F2S required at the time when the joining portion 40 is peeled off when the fastening tape 30 is pulled in the above-described predetermined direction. Specifically, the joining force in the joining portion 40 is adjusted such that, approximately, F1S ≥ 0.086 N/mm and 0.080 N/mm ≥ F2S ≥ 0.014 N/mm. Note that the reason that the magnitude of F2S has a certain range is that the magnitude of force required to peel off the joining portion 40 changes according to the direction in which the fastening tape 30 is pulled (any direction in the angle θ30 in FIG. 7B).

Accordingly, when the wearer who is wearing the diaper 1 moves their body or breathes, if the force F1 that acts when the fastening tape 30 is pulled outward in the width direction along the wearer's waist (i.e., the force that is naturally applied in the worn state) is smaller than 0.086 N/mm, there is a low possibility that the fastening tape 30 will be peeled off the side flap 14. However, when the diaper 1 is to be replaced, by pulling the inward end portion 30ei of the fastening tape with at least the force F2 of approximately 0.014 N/mm, the fastening tape 30 can be peeled off the side flap 14. Accordingly, spontaneous detachment of the fastening tape 30 in the worn state of the diaper 1 is suppressed, and the fastening tape 30 can be easily detached from the side flap 14 when removing the diaper 1.

Furthermore, in the present embodiment, the joining portion 40 is formed such that, when the diaper 1 is to be removed, the force F1S at the time when the joining portion 40 is peeled off when the region of the fastening tape 30 that is outward in the width direction with respect to the joining portion 40 is greater than the maximum value of the force F2S applied at the time when the joining portion 40 is peeled off when the region of the fastening tape 30 that is inward in the width direction with respect to the joining portion 40 (i.e., the inward end portion 30ei) is pulled. Accordingly, when removing the diaper 1, regardless of which direction within the angle θ30 shown in FIG. 7B the fastening tape 30 (inward end portion 30ei) is pulled, the fastening tape 30 can be easily detached from the side flap 14.

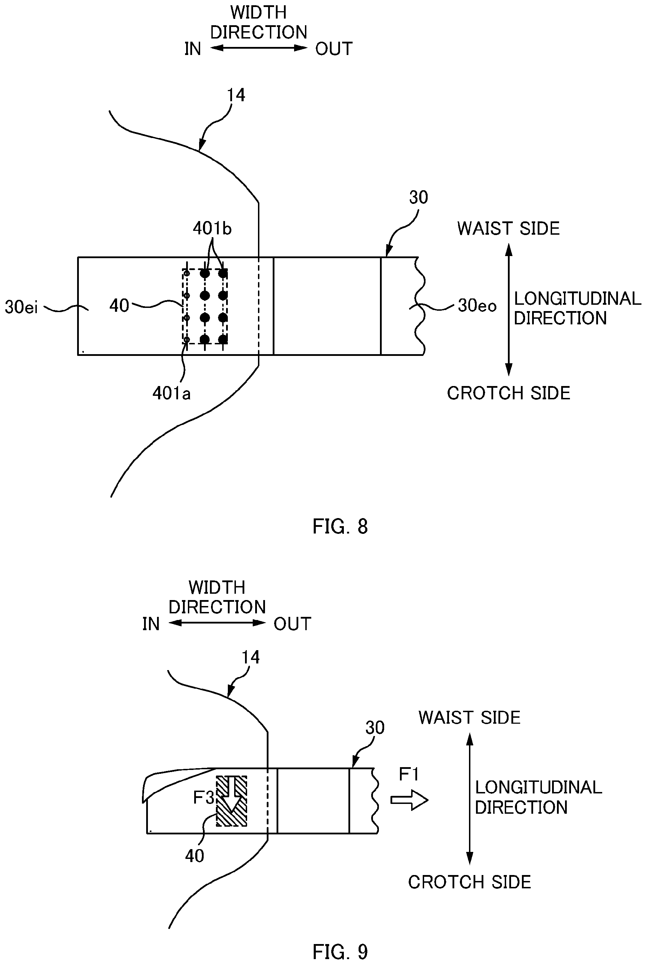

Also, the joining portion may be modified as described below such that the fastening tape 30 can be detached from the side flap 14 with a smaller magnitude of force when pulling the inward end portion 30ei of the fastening tape 30. FIG. 8 is a plan view illustrating a variation of the joining portion 40. In FIG. 8, similarly to FIG. 5, the joining portion 40 is formed by dot-like joining portions 401 that are arranged intermittently with three in each row in the width direction and four in each column in the longitudinal direction. Note that in the example in FIG. 8, among the dot-like joining portions 401, dot-like joining portions 401a that are arranged in the most inward column in the width direction (shown by white circles in FIG. 8) have a smaller area than dot-like joining portions 401b that are arranged in the other columns (shown by solid black circles in FIG. 8). In this case, the joining force of the fastening tape 30 and the side flap 14 exhibited by the dot-like joining portions 401a is weaker than the joining force exhibited by the dot-like joining portions 401b.

When the diaper 1 being worn by a low-weight infant is to be removed, if the inward end portion 30ei of the fastening tape 30 is pulled, the force F2 is first applied to the dot-like joining portions 401a that are arranged in the column that is the most inward in the width direction in the joining portion 40. Accordingly, if the joining strength exhibited by the dot-like joining portions 401 that are formed in the region where the force F2 acts first is set weaker than the joining strength exhibited by the dot-like joining portions 401 that are formed in the other regions, the fastening tape 30 can be peeled off more easily.

Also, in the case of patterns other than that shown in the variation in FIG. 8, it is possible to change the number of and shapes of the dot-like joining portions 401a that are arranged in the column that the most inward in the width direction among the dot-like joining portions 401. For example, a configuration is possible in which, by reducing the number of dot-like joining portions 401a arranged in each column in the longitudinal direction from four shown in FIG. 8 to three, and increasing the gap between the dot-like joining portions 401a that are adjacent in the longitudinal direction, the joining strength exhibited by the dot-like joining portions 401 in that region is reduced.

FIG. 7B illustrates the force F2 that acts in the case where the fastening tape 30 is pulled in any direction between outward in the width direction and outward in the thickness direction, but there are also cases where the fastening tape 30 is pulled in another direction. FIG. 9 is a diagram illustrating force acting on the joining portion 40 when the piece of fastening tape 30 is pulled in the longitudinal direction of the diaper 1. As shown in FIG. 9, if the region of the fastening tape 30 that is inward in the width direction with respect to the joining portion 40 (i.e., the inward end portion 30ei) is pulled from one side to the other side in the longitudinal direction (e.g., from the waist side toward the crotch side), force F3 along the longitudinal direction acts on the joining portion 40. It is assumed that when the magnitude of this force F3 reaches F3S, the joining portion 40 is peeled off, and the fastening tape 30 detaches from the side flap 14. Note that the magnitude of F3S is approximately 0.4 N/mm ≥ F3S ≥ 0.02 N/mm.

Accordingly, in the diaper 1, the force F1S required to peel off the joining portion 40 when the fastening tape 30 is pulled outward in the width direction is greater than the joining portion 40 is formed such that the force F3S required to peel off the joining portion 40 when the fastening tape 30 is pulled in the longitudinal direction. In this case, spontaneous detachment of the fastening tape 30 in the worn state of the diaper 1 is suppressed, and the fastening tape 30 can be easily detached from the side flap 14 by being pulled in the longitudinal direction.

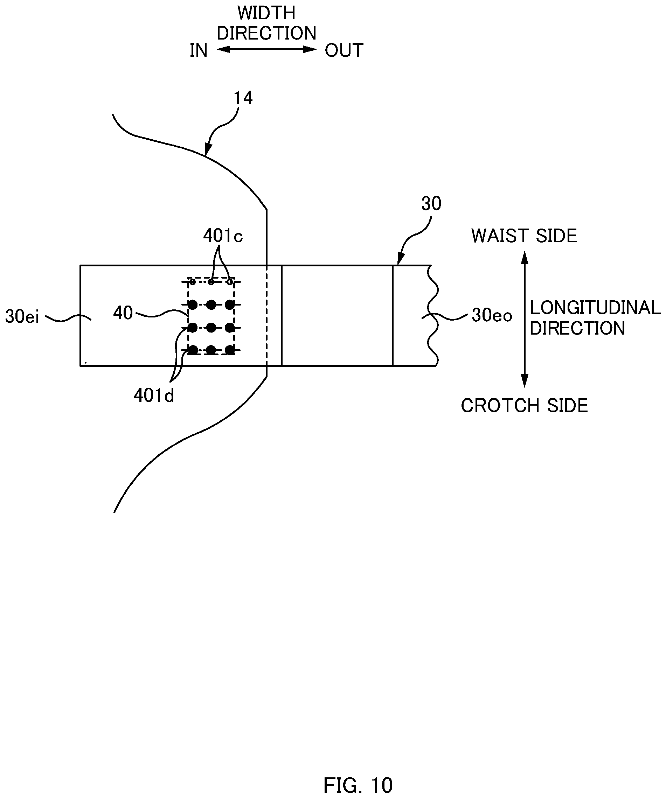

Also, the shape of the joining portion may be changed such that, when the fastening tape 30 is pulled in the longitudinal direction, the fastening tape 30 can be detached from the side flap 14 with a smaller magnitude of force. FIG. 10 is a plan view showing another variation of the joining portion 40. In the example in FIG. 10, among the dot-like joining portions 401, the area of dot-like joining portions 401c (shown by white circles in FIG. 10) that are arranged in the row that is the farthest on the waist side in the longitudinal direction is smaller than the area of dot-like joining portions 401d (shown by solid black circles in FIG. 10) that are arranged in the other rows. In this case, the joining force of the fastening tape 30 and the side flap 14 exhibited by the dot-like joining portions 401c is weaker than the joining force exhibited by the dot-like joining portions 401d. Accordingly, the fastening tape 30 can be more easily peeled off in the case where the fastening tape 30 is pulled from the waist side toward the crotch side in the longitudinal direction.

Also, in the case where the joining portion 40 is formed as shown in FIG. 10, the force required to peel off the joining portion 40 when the fastening tape 30 is pulled from the waist side toward the crotch side in the longitudinal direction is smaller than the force required to peel off the joining portion 40 when the fastening tape 30 is pulled from the crotch side toward the waist side in the longitudinal direction. In other words, the joining portion 40 is easily peeled off when the fastening tape 30 is pulled from the waist side toward the crotch side in the longitudinal direction, but the joining portion 40 is not easily peeled off when the fastening tape 30 is pulled from the crotch side toward the waist side in the longitudinal direction. This therefore suppresses problems such as the joining portion 40 being peeled off from the crotch side toward the waist side and the diaper 1 spontaneously coming off when the low-weight infant who is wearing the diaper 1 and is in the positioning posture moves their legs. However, when the joining portion 40 is to be peeled off in order to take off the diaper 1, the joining portion 40 can be easily peeled off by inserting a finger into the diaper and then pulling the fastening tape 30 in the direction from the waist side toward the crotch side.

Also, the force F2S and the force F3S applied at the time when the joining portion 40 is peeled off when the fastening tape 30 is pulled are greater than the force required to detach the engaging portion 31 of the fastening tape 30, which is engaged with the target tape 29, from the target tape 29 (target region) . Due to the engaging portion 31 having a hook-and-loop fastener structure or the like as described above, the engaging force is set such that the engaging portion 31 can be removably engaged with the target tape 29. This is because there is demand, when the diaper 1 is put on, for the ability to repeatedly attach and remove the engaging portion in order to adjust the fit around the waist and adjust the worn position of the diaper 1. However, the joining of the fastening tape 30 and the side flap 14 via the joining portion 40 need only be undone one time when taking off the diaper 1, and this joining is not repeatedly done and undone. Accordingly, in the diaper 1, the force F2S and the force F3S that are applied at the time when the joining portion 40 is peeled off when pulling the fastening tape 30 are set higher than the force applied for detaching the engaging portion 31 of the fastening tape 30 from the target region.

Also, when comparing the force F2S and the force F3S, F3S is smaller than F2S. Accordingly, when the fastening tape 30 is to be pulled away from the side flap 14, the fastening tape 30 can be more easily peeled away by pulling the inward end portion 30ei of the fastening tape in the longitudinal direction than in the width direction.

Also, when the fastening tape 30 is to be pulled off, the worker uses their fingers to grab a region of the fastening tape 30 that corresponds to a dry edge and that is inward in the width direction with respect to the region 304. Specifically, the worker uses one hand (fingers) to grab the region that is indicated by a distance h1 in FIG. 4 and that extends from the widthwise inward end of the fastening tape 30 to the widthwise inward end of the joining portion 40 (region 304). Here, in order to pull the fastening tape 30 and effectively apply force for peeling off the joining portion 40, it is desirable that the worker's other hand can securely grab the side flap 14 or the central band-shaped region 12 of the diaper 1. In the diaper 1, the fastening tape 30 is arranged such that a distance h2 from the longitudinal one-side end (waist-side end in FIG. 4) of the side flap 14 (central band-shaped region 12) to the longitudinal one-side end (waist-side end in FIG. 4) of the fastening tape 30 is greater than the distance h1.

According to this configuration, the worker can use one hand to pull the region of the fastening tape 30 indicated by the distance h1 (the region including the inward end portion 30ei) while using the other hand to securely grab the portion of the side flap 14 (central band-shaped region 12) indicated by the distance h2. Accordingly, the force pulling the fastening tape 30 is more easily applied to the joining portion 40, and even in the case of a small space such as in an incubator, the fastening tape 30 can be more easily detached from the side flap 14 without burdening the wearer's (low-weight infant's) body, and the diaper 1 can be replaced more easily.

Also, a configuration is possible in which an indicator mark is provided so as to indicate which portion is to be grabbed and pulled and when pulling the fastening tape 30. In FIG. 4, an indicator mark 50 that indicates the position of the inward end portion 30ei of the fastening tape 30 is provided on the outer surface of the side flap 14. By viewing this indicator mark 50, the worker can easily and accurately recognize which portion of the fastening tape 30 is to be pulled in order to easily peel off the joining portion 40. Note that the indicator mark 50 may be provided in another portion. For example, the indicator mark 50 may be provided on the surface of the fastening tape 30. Also, the indicator mark 50 may indicate the direction in which the fastening tape 30 is to be pulled.

### Variations

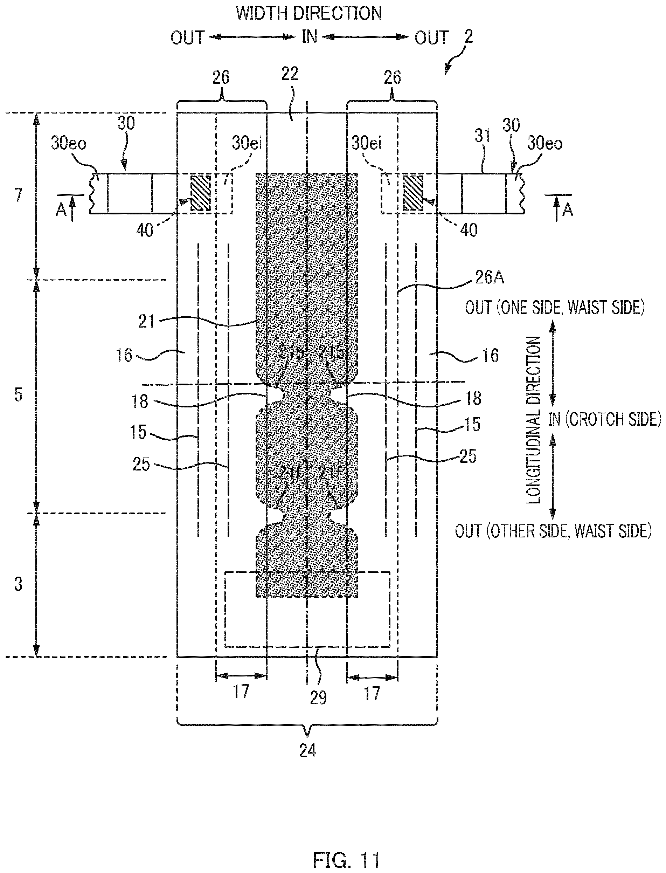

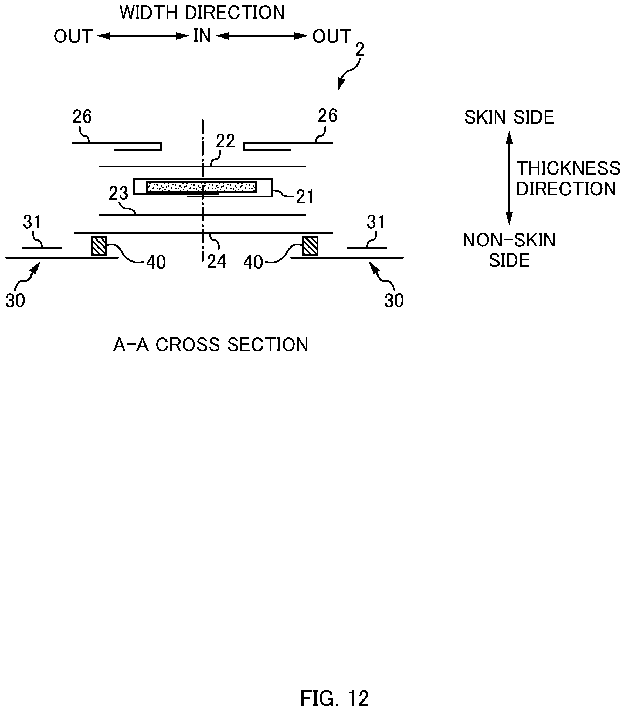

In the above embodiment, an example is described in which the side flaps 14 provided in the diaper 1 extend outward in the width direction on respective sides of the back portion 7, and the pieces of fastening tape 30 are joined to these extending portions via the joining portions 40, but the shape of the diaper 1 can also be modified as described below. FIG. 11 is a plan view showing a variation of the diaper 1 (diaper 2) in an unfolded and stretched state. FIG. 12 is an exploded illustrative view of the back portion 7 of the diaper 2 (A-A cross-section).

As shown in FIG. 11, in the diaper 2 of this variation, the skin-side sheet 26 and the back sheet 24 that constitute the side flaps 14 of the diaper 1 do not extend outward in the width direction in the back portion 7. Specifically, the two widthwise ends of the back sheet 24 (and skin-side sheet 26) that constitutes the exterior sheet of the diaper 2 are formed so as to be straight lines that extend in the longitudinal direction. And, with the exception of the pieces of fastening tape 30, the diaper 2 has a substantially rectangular shape. Because there are no portions that extend outward in the width direction, the overall width of the diaper 2 can be reduced. In addition, the diaper 2 itself can be used as an absorbent article such as a so-called absorbent pad or an absorbent liner. Note that other structures and functions of the diaper 2 other than the portions corresponding to the side flaps 14 (i.e., the back sheet 24 and the skin-side sheet 26) are substantially similar to those of the diaper 1 and therefore will not be described in detail.

In the back portion 7 of the diaper 2 according to this variation, the pieces of fastening tape 30 are respectively arranged on the two widthwise side portions of the exterior sheet (back sheet 24). Similarly to the diaper 1, the inward end portions 30ei of the pieces of fastening tape 30 have widthwise portions that are overlapped with the skin side in the thickness direction of the exterior sheet (back sheet 24) of the diaper 2. And the outward end portions 30eo of the pieces of fastening tape 30 have portions that project outward from respective ends of the exterior sheet (back sheet 24). Also, for each of the pieces of fastening tape 30, the joining portion 40 is formed in at least a portion of the region where the fastening tape 30 and the exterior sheet (back sheet 24) are overlapped in the thickness direction. The configuration of the joining portion 40 is similar to that of the diaper 1, and the joining portion 40 may be formed by a plurality of dot-like joining portions 401, for example.

In the state where a low-weight infant is wearing the diaper 2 and is in the positioning posture, when the worker performs the task of taking the diaper 2 off the wearer's body, similarly to the description given with reference to FIGS. 6A to 6C, the worker grabs the inward end portions 30ei of the pieces of fastening tape 30 and pulls them outward in the lateral direction or downward in the vertical direction so as to peel off the joining portions 40. This makes it possible to detach the pieces of fastening tape 30 from the exterior sheet (back sheet 24).

With the diaper 2, similarly to the diaper 1, the minimum value of the magnitude of the force F2 (see FIG. 7B) for pulling the region of the fastening tape 30 that is inward in the width direction with respect to the joining portion 40 (i.e., the region including the inward end portion 30ei) in any direction between the outward direction along the width direction and the outward direction along the thickness direction is smaller than the force F1 (see FIG. 7A) by which the region of the fastening tape 30 that is outward in the width direction with respect to the joining portion 40 (i.e., the region including the outward end portion 30eo) is pulled outward in the width direction.

Accordingly, when the diaper 2 is to be replaced, the fastening tape 30 can be peeled off the exterior sheet (back sheet 24) by pulling the inward end portion 30ei of the fastening tape with the force F2. Accordingly, spontaneous detachment of the fastening tape 30 in the worn state of the diaper 2 is suppressed, and the fastening tape 30 can be easily detached from the exterior sheet (back sheet 24) when removing the diaper 1.

Also, the joining portions 40 of the diaper 2 according to this variation have a similar configuration to the diaper 1 (see FIGS. 4 to 10), and therefore as described above, the joining portions 40 can be peeled off easily, and it is possible to reduce the burden on the wearer's body and the burden on the worker when removing the diaper 2.

### Other remarks

The above embodiments are for facilitating understanding of the present invention, and are not to be construed as limiting the present invention. The present invention may be changed or improved without departing from its gist, and, needless to say, encompasses equivalents thereof.

In the above embodiment, an example is described in which the target tape 29 has loops, the engaging portions 31 of the pieces of fastening tape 30 have hooks, and the engaging portions 31 are engaged with the target tape 29 by the hooks becoming caught on the loops, but the configurations of the target tape 29 and the engaging portions 31 are not limited to the example described above. For example, a configuration is possible in which the target tape 29 has hooks, the engaging portions 31 have loops, and the loops of the engaging portions 31 are locked to the hooks of the target tape 29. Also, a configuration is possible in which an adhesive member is provided on the outer surface of at least one out of the target tape 29 and the engaging portions 31, and these portions are locked to each other by the adhesive member being affixed to the outer surface of the other portion.

### [Reference Signs List]

- 1: disposable diaper (diaper),
- 2: disposable diaper (diaper),
- 3: front portion, 5 crotch portion, 7 back portion,
- 12: central band-shaped region,
- 14: side flap,
- 15: leg-gather elastic member,
- 16: leg gather,
- 17: leg side gather,
- 18: leg-side-gather elastic member,
- 21: absorbent body, 21f dart portion, 21b dart portion,
- 22: top sheet, 23 leak-proof sheet,
- 24: back sheet (exterior sheet)
- 25: leg elastic member,
- 26: skin-side sheet, 26A joining portion,
- 29: target tape,
- 30: fastening tape,
- 30ei: inward end portion, 30e outward end portion,
- 31: engaging portion,
- 40: joining portion,
- 50: indicator mark,
- 304: region,
- 401: dot-like joining portion, 401a, 401b, 401c, 401d dot-like joining portion,
- F1, F1S, F2, F2S, F3, F3S: force,
- θ30: angle

## Claims

1. A disposable diaper (1) having a longitudinal direction, a width direction, and a thickness direction that intersect each other,
the disposable diaper comprising:
side flaps (14);
pieces of fastening tape (30) respectively arranged on widthwise side portions of the side flaps (14); and
joining portions (40) each of which joins a portion of each piece of the fastening tape (30) to a non-skin side of the side flaps (14) in the thickness direction,
in an unfolded state, for each of the pieces of fastening tape (30),
a magnitude of force (F1S) applied at a time when the joining portion (40) is peeled off when a region of the fastening tape (30) that is outward in the width direction with respect to the joining portion (40) is pulled in an outward direction along the width direction
being greater than
a minimum value of an magnitude of force (F2S) applied at a time when the joining portion (40) is peeled off when a region of the fastening tape (30) that is inward in the width direction with respect to the joining portion (40) is pulled in any direction between the outward direction along the width direction and an outward direction along the thickness direction.

2. The disposable diaper according to claim 1, wherein
in the unfolded state, for each of the pieces of fastening tape (30),
the magnitude of force (F1S) applied at the time when the joining portion (40) is peeled off when the region of the fastening tape (30) that is outward in the width direction with respect to the joining portion (40) is pulled in the outward direction along the width direction is greater than
a maximum value of the magnitude of force (F2S) applied at the time when the joining portion (40) is peeled off when the region of the fastening tape (30) that is inward in the width direction with respect to the joining portion (40) is pulled in any direction between the outward direction along the width direction and the outward direction along the thickness direction.

3. The disposable diaper according to claim 1 or 2, wherein
for each of the pieces of fastening tape (30),
a widthwise stretchability in a widthwise region of the fastening tape that has a predetermined width and that includes the joining portion (40)
is less than or equal to
a widthwise stretchability in a region of the fastening tape (30) other than the widthwise region that has the predetermined width.

4. The disposable diaper according to claim 3, wherein
the widthwise region of the fastening tape (30) that has the predetermined width has no widthwise stretchability.

5. The disposable diaper according to any of claims 1 to 4, wherein
each of the joining portions (40) has a plurality of dot-like joining portions (401) that are arranged intermittently along the width direction, and
an area of a dot-like joining portion (401a) arranged at a position that is farthest inward in the width direction is smaller than an area of a dot-like joining portion (401b) arranged at another position.

6. The disposable diaper according to any of claims 1 to 5, wherein
in the unfolded state, for each of the pieces of fastening tape (30),
the magnitude of force (F1S) applied at a time when the joining portion is peeled off when the region of the fastening tape (30) that is outward in the width direction with respect to the joining portion (40) is pulled in the outward direction along the width direction is greater than
a minimum value of an magnitude of force (F3S) applied at a time when the joining portion (40) is peeled off when the region of the fastening tape (30) that is inward in the width direction with respect to the joining portion (40) is pulled from a waist side toward a crotch side along the longitudinal direction.

7. The disposable diaper according to claim 6, wherein
each of the joining portions (40) has a plurality of dot-like joining portions (401) that are arranged intermittently along the longitudinal direction, and
an area of a dot-like joining portion (401c) arranged at a position that is farthest on the waist side in the longitudinal direction is smaller than an area of a dot-like joining portion (401d) arranged at another position.

8. The disposable diaper according to claim 7, wherein
for each of the pieces of fastening tape (30),
the magnitude of force applied at a time when the joining portion (40) is peeled off when the region of the fastening tape (30) that is inward in the width direction with respect to the joining portion (40) is pulled from the waist side toward the crotch side along the longitudinal direction
is smaller than
an magnitude of force applied to peel off the joining portion (40) when the region of the fastening tape (30) that is inward in the width direction with respect to the joining portion (40) is pulled from the crotch side toward the waist side along the longitudinal direction.

9. The disposable diaper according to any of claims 1 to 8, wherein
for each of the pieces of fastening tape (30),
a longitudinal distance (h2) from a waist-side end of the side flap (14) to a waist-side end of the fastening tape (30)
is longer than
a widthwise distance (h1) from an inward end of the fastening tape (30) to an inward end of the joining portion (40).

10. The disposable diaper according to any of claims 1 to 9, wherein
the disposable diaper further comprises a target region (29) to which the pieces of fastening tape (30) are to be engaged, and
in the unfolded state, for each of the pieces of fastening tape (30),
the magnitude of force (F2S) applied at the time when the joining portion (40) is peeled off when the region of the fastening tape (29) that is inward in the width direction with respect to the joining portion (40) is pulled in any direction between the outward direction along the width direction and the outward direction along the thickness direction
is greater than
an magnitude of force applied at a time when the fastening tape (30) is detached from the target region (29) in a state where the fastening tape is engaged with the target region.

11. The disposable diaper according to any of claims 1 to 10, wherein
an indicator mark (50) is provided that indicates the region of the fastening tape (30) that is inward in the width direction with respect to the joining portion (40).

12. A disposable diaper (1) having a longitudinal direction, a width direction, and a thickness direction that intersect each other,
the disposable diaper comprising:
a liquid-absorbent absorbent body (21);
an exterior sheet (24) provided on a non-skin side in the thickness direction with respect to the absorbent body;
pieces of fastening tape (30) respectively arranged on widthwise side portions of the exterior sheet (24); and
joining portions (40) each of which joins a portion of each piece of the fastening tape (30) to a non-skin side of the exterior sheet (24) in the thickness direction,
in an unfolded state, for each of the pieces of fastening tape (30),
a magnitude of force (F1S) applied at a time when the joining portion (40) is peeled off when a region of the fastening tape (30) that is outward in the width direction with respect to the joining portion (40) is pulled in an outward direction along the width direction is greater than a minimum value of a magnitude of force (F2S) applied at a time when the joining portion (40) is peeled off when a region of the fastening tape (30) that is inward in the width direction with respect to the joining portion (40) is pulled in any direction between the outward direction along the width direction and an outward direction along the thickness direction.

## Patentansprüche

1. Einwegwindel (1), die eine Längsrichtung, eine Breitenrichtung und eine Dickenrichtung aufweist, die einander schneiden,
wobei die Einwegwindel Folgendes umfasst:
Seitenklappen (14);
Stücke des Befestigungsbands (30), die jeweils auf Breitenseitenteilen der Seitenklappen (14) angeordnet sind; und
Verbindungsteile (40), von denen jeder einen Teil von jedem Stück des Befestigungsbands (30) mit einer Nicht-Hautseite der Seitenklappe (14) in der Dickenrichtung verbindet,
wobei in einem aufgefalteten Zustand für jedes der Stücke des Befestigungsbands (30)
eine Größe einer Kraft (F1S), die zu einem Zeitpunkt angewendet wird, wenn der Verbindungsteil (40) abgelöst wird, wenn ein Bereich des Befestigungsbands (30), der sich außerhalb in der Breitenrichtung in Bezug auf den Verbindungsteil (40) befindet, in einer Richtung nach außen entlang der Breitenrichtung gezogen wird,
größer ist als
ein minimaler Wert einer Größe einer Kraft (F2S), die zu einem Zeitpunkt angewendet wird, wenn der Verbindungteil (40) abgelöst wird, wenn ein Bereich des Befestigungsbands (30), der sich innerhalb in der Breitenrichtung in Bezug auf den Verbindungsteil (40) befindet, in einer jedweden Richtung zwischen der Richtung nach außen entlang der Breitenrichtung und einer Richtung nach außen entlang der Dickenrichtung gezogen wird.

2. Einwegwindel nach Anspruch 1, wobei
in einem aufgefalteten Zustand für jedes der Stücke des Befestigungsbands (30)
die Größe einer Kraft (F1S), die zu dem Zeitpunkt angewendet wird, wenn der Verbindungsteil (40) abgelöst wird, wenn der Bereich des Befestigungsbands (30), der sich außerhalb in der Breitenrichtung in Bezug auf den Verbindungsteil (40) befindet, in der Richtung nach außen entlang der Breitenrichtung gezogen wird,
größer ist als
ein maximaler Wert der Größe einer Kraft (F2S), die zu dem Zeitpunkt angewendet wird, wenn der Verbindungteil (40) abgelöst wird, wenn der Bereich des Befestigungsbands (30), der sich innerhalb in der Breitenrichtung in Bezug auf den Verbindungsteil (40) befindet, in einer jedweden Richtung zwischen der Richtung nach außen entlang der Breitenrichtung und der Richtung nach außen entlang der Dickenrichtung gezogen wird.

3. Einwegwindel nach Anspruch 1 oder 2, wobei
für jedes der Stücke des Befestigungsbands (30)
eine Breitendehnbarkeit in einem Breitenbereich des Befestigungsbands, der eine vorgegebene Breite aufweist und der den Verbindungsteil (40) einschließt,
kleiner als oder gleich
einer Breitendehnbarkeit in einem Bereich des Befestigungsbands (30) ist, der nicht der Breitenbereich ist, der die vorgegebene Breite aufweist.

4. Einwegwindel nach Anspruch 3, wobei
der Breitenbereich des Befestigungsbands (30), der die vorgegebene Breite aufweist, keine Breitendehnbarkeit aufweist.

5. Einwegwindel nach einem der Ansprüche 1 bis 4, wobei
jeder der Verbindungsteile (40) eine Vielzahl von punktförmigen Verbindungsteilen (401) aufweist, die entlang der Breitenrichtung intermittierend angeordnet sind, und
eine Fläche eines punktförmigen Verbindungsteils (401a), der an einer Position angeordnet ist, die sich am weitesten nach innen in der Breitenrichtung befindet, kleiner ist als eine Fläche eines punktförmigen Verbindungsteils (401b), der an einer anderen Position angeordnet ist.

6. Einwegwindel nach einem der Ansprüche 1 bis 5, wobei
in einem aufgefalteten Zustand für jedes der Stücke des Befestigungsbands (30)
die Größe einer Kraft (F1S), die zu einem Zeitpunkt angewendet wird, wenn der Verbindungsteil abgelöst wird, wenn der Bereich des Befestigungsbands (30), der sich außerhalb in der Breitenrichtung in Bezug auf den Verbindungsteil (40) befindet, in einer Richtung nach außen entlang der Breitenrichtung gezogen wird,
größer ist als
ein minimaler Wert einer Größe einer Kraft (F3S), die zu einem Zeitpunkt angewendet wird, wenn der Verbindungteil (40) abgelöst wird, wenn der Bereich des Befestigungsbands (30), der sich innerhalb in der Breitenrichtung in Bezug auf den Verbindungsteil (40) befindet, von einer Taillenseite in Richtung einer Schrittseite entlang der Längsrichtung gezogen wird.

7. Einwegwindel nach Anspruch 6, wobei
jeder der Verbindungsteile (40) eine Vielzahl von punktförmigen Verbindungsteilen (401) aufweist, die entlang der Längsrichtung intermittierend angeordnet sind, und
eine Fläche eines punktförmigen Verbindungsteils (401c), der an einer Position angeordnet ist, die sich am weitesten auf der Taillenseite in der Längsrichtung befindet, kleiner ist als eine Fläche eines punktförmigen Verbindungsteils (401d), der an einer anderen Position angeordnet ist.

8. Einwegwindel nach Anspruch 7, wobei
für jedes der Stücke des Befestigungsbands (30)
die Größe einer Kraft, die zu einem Zeitpunkt angewendet wird, wenn der Verbindungsteil (40) abgelöst wird, wenn der Bereich des Befestigungsbands (30), der sich innerhalb in der Breitenrichtung in Bezug auf den Verbindungsteil (40) befindet, von der Taillenseite in Richtung der Schrittseite entlang der Längsrichtung gezogen wird,
kleiner ist als
eine Größe einer Kraft, die angewendet wird, um den Verbindungteil (40) abzulösen, wenn der Bereich des Befestigungsbands (30), der sich innerhalb in der Breitenrichtung in Bezug auf den Verbindungsteil (40) befindet, von der Schrittseite in Richtung der Taillenseite entlang der Längsrichtung gezogen wird.

9. Einwegwindel nach einem der Ansprüche 1 bis 8, wobei für jedes der Stücke des Befestigungsbands (30)
ein Längsabstand (h2) von einem Taillenseitenende der Seitenklappe (14) zu einem Taillenseitenende des Befestigungsbands (30)
länger ist als
ein Breitenabstand (h1) von einem inneren Ende des Befestigungsbands (30) zu einem inneren Ende des Verbindungsteils (40).

10. Einwegwindel nach einem der Ansprüche 1 bis 9, wobei
die Einwegwindel weiter einen Zielbereich (29) umfasst, in den die Stücke des Befestigungsbands (30) eingreifen sollen, und
in dem aufgefalteten Zustand für jedes der Stücke des Befestigungsbands (30)
die Größe einer Kraft (F2S), die zu dem Zeitpunkt angewendet wird, wenn der Verbindungsteil (40) abgelöst wird, wenn der Bereich des Befestigungsbands (29), der sich innerhalb in der Breitenrichtung in Bezug auf den Verbindungsteil (40) befindet, in einer jedweden Richtung zwischen der Richtung nach außen entlang der Breitenrichtung und der Richtung nach außen entlang der Dickenrichtung gezogen wird,
größer ist als
eine Größe einer Kraft, die zu einem Zeitpunkt angewendet wird, wenn das Befestigungsband (30) von dem Zielbereich (29) in einem Zustand abgetrennt wird, wo das Befestigungsband in den Zielbereich eingreift.

11. Einwegwindel nach einem der Ansprüche 1 bis 10, wobei
eine Kennzeichnungsmarkierung (50) bereitgestellt ist, die den Bereich des Befestigungsbands (30) kennzeichnet, der innerhalb in der Breitenrichtung in Bezug auf den Verbindungsteil (40) vorliegt.

12. Einwegwindel (1), die eine Längsrichtung, eine Breitenrichtung und eine Dickenrichtung aufweist, die einander schneiden,
wobei die Einwegwindel Folgendes umfasst:
einen Flüssigkeit absorbierenden Saugkörper (21);
eine äußere Lage (24), die auf einer Nicht-Hautseite in der Dickenrichtung in Bezug auf den Saugkörper bereitgestellt ist;
Stücke des Befestigungsbands (30), die jeweils auf Breitenseitenteilen der äußeren Lage (24) angeordnet sind; und
Verbindungsteile (40), von denen jeder einen Teil von jedem Stück des Befestigungsbands (30) mit einer Nicht-Hautseite der äußeren Lage (24) in der Dickenrichtung verbindet,
wobei in einem aufgefalteten Zustand für jedes der Stücke des Befestigungsbands (30)
eine Größe einer Kraft (F1S), die zu einem Zeitpunkt angewendet wird, wenn der Verbindungsteil (40) abgelöst wird, wenn ein Bereich des Befestigungsbands (30), der sich außerhalb in der Breitenrichtung in Bezug auf den Verbindungsteil (40) befindet, in einer Richtung nach außen entlang der Breitenrichtung gezogen wird, größer ist als ein minimaler Wert einer Größe einer Kraft (F2S), die zu einem Zeitpunkt angewendet wird, wenn der Verbindungteil (40) abgelöst wird, wenn ein Bereich des Befestigungsbands (30), der sich innerhalb in der Breitenrichtung in Bezug auf den Verbindungsteil (40) befindet, in einer jedweden Richtung zwischen der Richtung nach außen entlang der Breitenrichtung und einer Richtung nach außen entlang der Dickenrichtung gezogen wird.

## Revendications

1. Couche jetable (1) ayant une direction allant dans le sens longitudinal, une direction allant dans le sens de la largeur, et une direction allant dans le sens de l'épaisseur qui se croisent les unes les autres,
la couche jetable comportant :
des rabats latéraux (14) ;
des morceaux de ruban d'attache (30) agencés respectivement sur des parties latérales dans le sens de la largeur des rabats latéraux (14) ; et
des parties d'assemblage (40) dont chacune assemble une partie de chaque morceau du ruban d'attache (30) à un côté non orienté vers la peau des rabats latéraux (14) dans la direction allant dans le sens de l'épaisseur,
dans un état déplié, pour chacun des morceaux de ruban d'attache (30),
une amplitude de la force (F1S) appliquée à un moment où la partie d'assemblage (40) est décollée quand une région du ruban d'attache (30) qui se trouve vers l'extérieur dans la direction allant dans le sens de la largeur par rapport à la partie d'assemblage (40) est tirée dans une direction allant vers l'extérieur le long de la direction allant dans le sens de la largeur
étant supérieure à
une valeur minimum d'une amplitude de la force (F2S) appliquée à un moment où la partie d'assemblage (40) est décollée quand une région du ruban d'attache (30) qui se trouve vers l'intérieur dans la direction allant dans le sens de la largeur par rapport à la partie d'assemblage (40) est tirée dans une direction quelconque entre la direction allant vers l'extérieur le long de la direction allant dans le sens de la largeur et une direction allant vers l'extérieur le long de la direction allant dans le sens de l'épaisseur.

2. Couche jetable selon la revendication 1, dans laquelle
dans l'état déplié, pour chacun des morceaux de ruban d'attache (30),
l'amplitude de la force (F1S) appliquée au moment où la partie d'assemblage (40) est décollée quand la région du ruban d'attache (30) qui se trouve vers l'extérieur dans la direction allant dans le sens de la largeur par rapport à la partie d'assemblage (40) est tirée dans la direction allant vers l'extérieur le long de la direction allant dans le sens de la largeur
est supérieure à
une valeur maximum de l'amplitude de la force (F2S) appliquée au moment où la partie d'assemblage (40) est décollée quand la région du ruban d'attache (30) qui se trouve vers l'intérieur dans la direction allant dans le sens de la largeur par rapport à la partie d'assemblage (40) est tirée dans une direction quelconque entre la direction allant vers l'extérieur le long de la direction allant dans le sens de la largeur et la direction allant vers l'extérieur le long de la direction allant dans le sens de l'épaisseur.

3. Couche jetable selon la revendication 1 ou la revendication 2, dans laquelle
pour chacun des morceaux de ruban d'attache (30),
une extensibilité dans le sens de la largeur dans une région dans le sens de la largeur du ruban d'attache qui a une largeur prédéterminée et qui comprend la partie d'assemblage (40)
est inférieure ou égale à
une extensibilité dans le sens de la largeur dans une région du ruban d'attache (30) autre que la région dans le sens de la largeur qui a la largeur prédéterminée.

4. Couche jetable selon la revendication 3, dans laquelle
la région dans le sens de la largeur du ruban d'attache (30) qui a la largeur prédéterminée n'a aucune extensibilité dans le sens de la largeur.

5. Couche jetable selon l'une quelconque des revendications 1 à 4, dans laquelle
chacune des parties d'assemblage (40) a une pluralité de parties d'assemblage similaires à des points (401) qui sont agencées de manière intermittente le long de la direction allant dans le sens de la largeur, et
une zone de la partie d'assemblage similaire à un point (401a) agencée au niveau d'une position qui se trouve au plus loin vers l'intérieur dans la direction allant dans le sens de la largeur est inférieure à une zone d'une partie d'assemblage similaire à un point (401b) agencée au niveau d'une autre position.

6. Couche jetable selon l'une quelconque des revendications 1 à 5, dans laquelle dans l'état déplié, pour chacun des morceaux de ruban d'attache (30),
l'amplitude de la force (F1S) appliquée à un moment où la partie d'assemblage est décollée quand la région du ruban d'attache (30) qui se trouve vers l'extérieur dans la direction allant dans le sens de la largeur par rapport à la partie d'assemblage (40) est tirée dans la direction allant vers l'extérieur le long de la direction allant dans le sens de la largeur
est supérieure à
une valeur minimum d'une amplitude de la force (F3S) appliquée à un moment où la partie d'assemblage (40) est décollée quand la région du ruban d'attache (30) qui se trouve vers l'intérieur dans la direction allant dans le sens de la largeur par rapport à la partie d'assemblage (40) est tirée depuis un côté au niveau de la taille jusqu'à un côté au niveau de l'entrejambe le long de la direction allant dans le sens longitudinal.

7. Couche jetable selon la revendication 6, dans laquelle
chacune des parties d'assemblage (40) a une pluralité de parties d'assemblage similaires à des points (401) qui sont agencées de manière intermittente le long de la direction allant dans le sens longitudinal, et
une zone d'une partie d'assemblage similaire à un point (401c) agencée au niveau d'une position qui se trouve au plus loin sur le côté au niveau de la taille dans la direction allant dans le sens longitudinal est inférieure à une zone d'une partie d'assemblage similaire à un point (401d) agencée au niveau d'une autre position.

8. Couche jetable selon la revendication 7, dans laquelle
pour chacun des morceaux de ruban d'attache (30),
l'amplitude de la force appliquée à un moment où la partie d'assemblage (40) est décollée quand la région du ruban d'attache (30) qui se trouve vers l'intérieur dans la direction allant dans le sens de la largeur par rapport à la partie d'assemblage (40) est tirée depuis le côté au niveau de la taille vers le côté au niveau de l'entrejambe le long de la direction allant dans le sens longitudinal
est inférieure à
une amplitude de la force appliquée pour tirer la partie d'assemblage (40) quand la région du ruban d'attache (30) qui se trouve vers l'intérieur dans la direction allant dans le sens de la largeur par rapport à la partie d'assemblage (40) est tirée depuis le côté au niveau de l'entrejambe vers le côté au niveau de la taille dans la direction allant dans le sens longitudinal.

9. Couche jetable selon l'une quelconque des revendications 1 à 8, dans laquelle
pour chacun des morceaux de ruban d'attache (30),
une distance longitudinale (h2) depuis une extrémité côté au niveau de la taille du rabat latéral (14) jusqu'à une extrémité côté au niveau de la taille du ruban d'attache (30)
est plus longue que
une distance dans le sens de la largeur (h1) depuis une extrémité vers l'intérieur du ruban d'attache (30) jusqu'à une extrémité vers l'intérieur de la partie d'assemblage (40).

10. Couche jetable selon l'une quelconque des revendications 1 à 9, dans laquelle
la couche jetable comporte par ailleurs une région cible (29) au niveau de laquelle les morceaux de ruban d'attache (30) sont destinés à être mis en prise, et
dans l'état déplié, pour chacun des morceaux de ruban d'attache (30),
l'amplitude de la force (F2S) appliquée au moment où la partie d'assemblage (40) est décollée quand la région du ruban d'attache (29) qui se trouve vers l'intérieur dans la direction allant dans le sens de la largeur par rapport à la partie d'assemblage (40) est tirée dans une direction quelconque entre la direction allant vers l'extérieur le long de la direction allant dans le sens de la largeur et la direction allant vers l'extérieur le long de la direction allant dans le sens de l'épaisseur
est supérieure à
une amplitude de la force appliquée à un moment où le ruban d'attache (30) est détaché de la région cible (29) dans un état dans lequel le ruban d'attache est mis en prise avec la région cible.

11. Couche jetable selon l'une quelconque des revendications 1 à 10, dans laquelle un repère indicateur (50) est mis en œuvre pour indiquer la région du ruban d'attache (30) qui se trouve vers l'intérieur dans la direction allant dans le sens de la largeur par rapport à la partie d'assemblage (40).

12. Couche jetable (1) ayant une direction allant dans le sens longitudinal, une direction allant dans le sens de la largeur, et une direction allant dans le sens de l'épaisseur qui se croisent les unes les autres,
la couche jetable comportant :
un corps absorbant servant à absorber les liquides (21) ;
une feuille extérieure (24) mise en œuvre sur un côté non orienté vers la peau dans la direction allant dans le sens de l'épaisseur par rapport au corps absorbant ;
des morceaux de ruban d'attache (30) agencés respectivement sur des parties latérales dans le sens de la largeur de la feuille extérieure (24) ; et
des parties d'assemblage (40) dont chacune assemble une partie de chaque morceau du ruban d'attache (30) à un côté non orienté vers la peau de la feuille extérieure (24) dans la direction allant dans le sens de l'épaisseur,
dans un état déplié, pour chacun des morceaux de ruban d'attache (30),
une amplitude de la force (F1S) appliquée à un moment où la partie d'assemblage (40) est décollée quand une région du ruban d'attache (30) qui se trouve vers l'extérieur dans la direction allant dans le sens de la largeur par rapport à la partie d'assemblage (40) est tirée dans une direction allant vers l'extérieur le long de la direction allant dans le sens de la largeur est supérieure à une valeur minimum d'une amplitude de la force (F2S) appliquée à un moment où la partie d'assemblage (40) est décollée quand une région du ruban d'attache (30) qui se trouve vers l'intérieur dans la direction allant dans le sens de la largeur par rapport à la partie d'assemblage (40) est tirée dans une direction quelconque entre la direction allant vers l'extérieur le long de la direction allant dans le sens de la largeur et une direction allant vers l'extérieur le long de la direction allant dans le sens de l'épaisseur.
